# EUROPÄISCHE PATENTANMELDUNG

(11) **EP 4 803 894 A1**
(43) Veröffentlichungstag der Anmeldung: **09.09.2026**
(21) Anmeldenummer: 26160731.1
(22) Anmeldetag: 25.02.2026
(51) Int. Cl.: G01N 27/28, G01N 27/416, G01N 33/18

(54) **ELEKTROCHEMISCHER SENSOR, MESSEINRICHTUNG MIT EINEM SOLCHEN SENSOR SOWIE VERFAHREN ZUR HERSTELLUNG EINES ELEKTROCHEMISCHEN SENSORS**

(30) Priorität: 07.03.2025 DE 102025108802
(71) Anmelder: ecm GmbH, 69469 Weinheim (DE)
(72) Erfinder: Reiß, Gerhard, 69469 Weinheim (DE); Starker, Christian, 69253 Heiligkreuzsteinach (DE); Zickler, Martin, 69469 Weinheim (DE); Kamke, Alexander, 67065 Ludwigshafen am Rhein (DE)
(74) Vertreter: Reiser & Partner Patentanwälte mbB

(57) **Zusammenfassung**

Die Erfindung bezieht sich auf einen elektrochemischen Sensor mit einer ersten Elektrode (3) und einer zweiten Elektrode (4), mit einem Träger (2), auf dem zumindest die erste Elektrode (3) angeordnet ist, und mit einer Poren aufweisenden Membran (9). Der Sensor ist dadurch gekennzeichnet, dass die Membran (9) an dem Träger (2) angeordnet ist und die erste Elektrode (3) überdecket, wobei die Membran (9) über eine Verbindungsschicht (14) mit dem Träger (2) verbunden ist, wobei die Verbindungsschicht (14) in dem Bereich der ersten Elektrode (3) eine erste Aussparung (15) aufweist, um über der ersten Elektrode (3) eine erste Messkammer (18) zu bilden. Die Erfindung bezieht sich darüber hinaus auf eine Messeinrichtung mit einem solchen Sensor und auf ein Verfahren zur Herstellung eines solchen Sensors.

## Beschreibung

Die Erfindung betrifft einen elektrochemischen Sensor mit einer ersten Elektrode und einer zweiten Elektrode, mit einem Träger, auf dem zumindest die erste Elektrode angeordnet ist und mit einer Poren aufweisenden Membran. Die Erfindung betrifft darüber hinaus ein Verfahren zur Herstellung eines solchen elektrochemischen Sensors.

Ein solcher elektrochemischer Sensor kann insbesondere dazu dienen, in einer Flüssigkeit und insbesondere in einer wässrigen Flüssigkeit ein Messsignal zu erzeugen, das von der Konzentration eines Stoffes in der Flüssigkeit abhängt.

Derartige elektrochemische Sensoren mit zwei oder mehr Elektroden sind bekannt. Sie können insbesondere für die elektrochemische amperometrische Chlor-, Chlordioxid-, Ozon-, Peressigsäure- und Wasserstoffperoxidmessung im Wasser verwendet werden.

Die DE 42 11 198 C2 beschreibt eine elektrochemische Messzelle zum Nachweis einer in Wasser gelösten Spezies mit drei Elektroden. Die geschlossen ausgebildete Messzelle weist einen Elektrolytraum auf, der von einer Membran begrenzt ist. Der Elektrolytraum ist mit Elektrolyten gefüllt, der eine Hilfssubstanz aufweist. Eine der Elektroden ist dabei in dem Elektrolytraum angeordnet. Nachteilig für die Handhabung ist bei dieser Gestaltung, dass die Messzelle mit einem gesonderten Elektrolyten zu befüllen ist. Dabei ist eine regelmäßige Wartung mit einer Erneuerung des Elektrolyten erforderlich.

Weiterhin sind offene Messzellen bekannt, die keine Membran aufweisen. Hier ist nachteilig, dass diese bei der Messung eine sehr konstante Anströmung und einen freien Messwasserauslauf benötigen.

Die US 2023/0194458 A1 beschreibt ein Verfahren zur Herstellung eines elektrochemischen Sensors, bei dem die Elektroden auf ein Trägermaterial aufgedruckt werden.

Die Erfindung stellt sich die Aufgabe, einen elektrochemischen Sensor anzugeben, der eine zuverlässige Messung ermöglicht und kostengünstig herstellbar ist.

Die Aufgabe wird durch einen elektrochemischen Sensor nach Anspruch 1 gelöst. Demnach ist ein elektrochemischer Sensor mit einer ersten Elektrode und einer zweiten Elektrode, mit einem Träger, auf dem zumindest die erste Elektrode angeordnet ist, und mit einer Poren aufweisenden Membran vorgesehen, wobei die Membran an dem Träger angeordnet ist und die erste Elektrode überdeckt, wobei die Membran über eine Verbindungsschicht mit Trägern verbunden ist, wobei die Verbindungsschicht in dem Bereich der ersten Elektrode eine erste Aussparung aufweist, um über der ersten Elektrode eine erste Messkammer zu bilden.

Ein solcher elektrochemischer Sensor ermöglicht es, Messungen mit hoher Genauigkeit durchzuführen. Dabei kann ein Messsignal erhalten werden, das das Vorhandensein und/oder die Konzentration eines oder mehrerer Stoffe in der Flüssigkeit anzeigt. Der Sensor eignet sich insbesondere für die amperometrische Messung in Flüssigkeiten, insbesondere in Wasser. Insbesondere eignet sich der Sensor für die Messung von chlorhaltigen Stoffen in wässrigen Flüssigkeiten. Besonders geeignet ist der Sensor für die Messung von freiem Chlor, Chloraminen, Chlordioxid, Ozon, Wasserstoffperoxid, Brom und/oder Chlorit in wässrigen Flüssigkeiten. Der elektrochemische Sensor weist eine hohe Lebensdauer auf und liefert über seine Betriebsdauer ein zuverlässiges Messsignal. Zudem liefert der Sensor auch bei wechselnder Anströmgeschwindigkeit der Flüssigkeit zuverlässige Werte. Darüber hinaus ist der Wartungsaufwand gering. Insbesondere ist es nicht erforderlich, einen spezifischen Elektrolyten hinzuzufügen und zu erneuern. Der elektrochemische Sensor ist auch gut geschützt gegen Verschmutzungen. Darüber hinaus ist der elektrochemische Sensor einfach und mit hoher Prozesssicherheit herstellbar.

Nachfolgend werden weitere Merkmale des elektrochemischen Sensors beschrieben, die die vorgenannten Vorteile und Wirkungen weiter fördern.

Für die zweite Elektrode kann ein zweiter Träger vorgesehen sein. Dieser kann im Hinblick auf die Anordnung der Elektrode, der Membran und der Verbindungsschicht dem ersten Träger entsprechen.

Eine bevorzugte Ausgestaltung der Erfindung sieht vor, dass auf dem Träger die zweite Elektrode angeordnet ist, wobei die Membran die zweite Elektrode überdeckt, wobei die Verbindungsschicht in dem Bereich der zweiten Elektrode eine zweite Aussparung aufweist, um über der zweiten Elektrode eine zweite Messkammer zu bilden. Mit dieser Gestaltung können besonders gute Messergebnisse erzielt werden. Zudem kann auf diese Weise ein kompakter Sensor mit mehreren Elektroden auf einem einzigen Träger bereitgestellt werden.

Eine weitere Verbesserung sieht vor, dass die Messeinrichtung eine dritte Elektrode umfasst. Um einen Strom durch eine elektrochemische Zelle fließen zu lassen, sind bereits zwei Elektroden ausreichend. Mit der Zwei-Elektroden-Anordnung können der Strom und die Zellspannung gemessen oder eingestellt werden. Die dritte Elektrode ermöglicht es insbesondere, das Elektrodenpotential an der Arbeitselektrode unabhängig von einem fließenden Strom zu messen. Die Messung dieses Potentiales ist vorteilhaft, weil es Einfluss auf die Elektrodenreaktion hat.

Es kann ein dritter Träger für die dritte Elektrode vorgesehen sein, der im Hinblick auf die Anordnung der Elektrode, der Membran und der Verbindungsschicht dem ersten Träger entspricht.

Erfindungsgemäß ist bevorzugt, dass auf dem Träger die dritte Elektrode angeordnet ist, wobei die Membran die dritte Elektrode überdeckt, wobei die Verbindungsschicht in dem Bereich der dritten Elektrode eine dritte Aussparung aufweist, um über der dritten Elektrode eine dritte Messkammer zu bilden. Das trägt zu einer weiteren Verbesserung der Messergebnisse bei. Zudem kann auf diese Weise ein kompakter Sensor mit drei Elektroden auf einem einzigen Träger bereitgestellt werden.

Eine bevorzugte Ausführung sieht vor, dass die erste und die zweite Messkammer gegeneinander abgedichtet sind. Bei der Ausführung mit drei Elektroden kann vorgesehen sein, dass die erste, zweite und dritte Messkammer gegeneinander abgedichtet ist. Auf diese Weise ergeben sich separate Messkammern, welche die Messgenauigkeit verbessern. Das wird darauf zurückgeführt, dass Signalstörungen durch Querströme und Ionenströme reduziert oder vermieden werden können.

Vorzugsweise hat die erste Messkammer eine rechteckige Form. Erfindungsgemäß kann vorgesehen sein, dass die zweite Messkammer und/oder die dritte Messkammer eine rechteckige Form aufweisen.

Die erste Elektrode, die zweite Elektrode und ggf. die dritte Elektrode können streifenförmig und/oder länglich ausgebildet sein. Vorzugsweise sind die erste, die zweite und die dritte Elektrode parallel zueinander angeordnet.

Eine erfindungsgemäße Ausführungsform sieht vor, dass die erste und die zweite Elektrode gleich groß sind. Das trägt dazu bei, genaue Messergebnisse zu erhalten.

Erfindungsgemäß ist besonders bevorzugt, wenn die erste Elektrode und die zweite Elektrode auf dem Träger angeordnet sind. Vorzugsweise ist bei der Ausführung mit drei Elektroden auch die dritte Elektrode auf dem Träger angeordnet.

Die Elektroden können in Dünnschichttechnik, wie z. B. mit dem PVD-Verfahren, dem Sputterverfahren und/oder dem Ionenstrahlverfahren aufgebracht sein.

Die Elektroden können auch in anderen Verfahren auf den Träger aufgebracht sein. Dabei ist erfindungsgemäß bevorzugt, wenn die Elektroden auf den Träger aufgedruckt sind. Hierzu können Pasten und Tinten verwendet werden, die einen leitfähigen Stoff enthalten. Eine bevorzugte Ausgestaltung sieht vor, dass die Elektroden im Siebdruckverfahren aufgebracht sind. Weitere geeignete Verfahren sind das Jetting, der Tampondruck und das Rolle-zu-Rolle-Verfahren.

Vorzugsweise werden die Elektroden durch eine vernetzte Polymermasse, die Metallpulver enthält, gebildet. Die vernetzte Polymermasse kann als Polymerpaste auf den Träger aufgebracht und anschließend vernetzt sein.

Vorzugsweise bildet die erste Elektrode eine Arbeitselektrode oder Messelektrode. Die Arbeits- oder Messelektrode enthält vorzugsweise ein Metall, insbesondere Gold, Platin, Palladium. Platin ist dabei besonders bevorzugt.

Vorzugsweise bildet die zweite Elektrode eine Gegenelektrode. Die Gegenelektrode: enthält vorzugsweise ein Metall, insbesondere Gold, Platin, Palladium. Platin ist dabei besonders bevorzugt.

Vorzugsweise bildet die dritte Elektrode eine Bezugselektrode. Die Bezugselektrode enthält vorzugsweise ein Metall, insbesondere Silber und/oder Silberhalogenid. Das Silberhalogenid kann insbesondere Silberchlorid, Silberbromid oder Silberjodid sein.

Erfindungsgemäß kann vorgesehen sein, dass die erste Aussparung nur einen Teil der ersten Elektrode frei lässt. Damit kann die für die Messung wirksame Elektrodenfläche der ersten Elektrode definiert werden. Das ist vorteilhaft für die Messungen mit dem Sensor. Insbesondere wird ein schnellerer Einlauf des Sensors erreicht, sodass der Sensor nach kürzerer Zeit zuverlässige Messwerte liefern kann.

Eine weitere Verbesserung sieht vor, dass die zweite Aussparung nur einen Teil der zweiten Elektrode frei lässt. Damit kann die für die Messung wirksame Elektrodenfläche der zweiten Elektrode definiert werden.

Erfindungsgemäß ist besonders bevorzugt, dass die dritte Aussparung nur einen Teil der dritten Elektrode frei lässt. Damit kann die für die Messung wirksame Elektrodenfläche der dritten Elektrode definiert werden.

Erfindungsgemäß kann vorgesehen sein, dass die erste Messkammer eine andere Größe als die zweite und/oder die dritte Messkammer aufweist. Besonders bevorzugt ist die erste Messkammer kleiner als die zweite Messkammer und die dritte Messkammer. Auf diese Weise kann mit der Verbindungsschicht die aktive Fläche der jeweiligen Elektrode unterschiedlich definiert werden. In bevorzugter Weise ist die aktive Fläche der ersten Elektrode kleiner als die aktive Fläche der zweiten und als die aktive Fläche der dritten Elektrode.

Eine bevorzugte Ausgestaltung sieht vor, dass die erste Aussparung eine Fläche zwischen 1 mm² und 40 mm² umfasst, vorzugsweise zwischen 3 mm² und 9 mm².

In vorteilhafter Weise kann vorgesehen sein, dass die zweite Aussparung eine Fläche zwischen 1 mm² und 40 mm² (vorzugsweise zwischen 3 mm² und 9 mm²) und/oder die dritte Aussparung eine Fläche zwischen 1 und 40 mm² (vorzugsweise zwischen 3 mm² und 9 mm²) umfasst.

Vorzugsweise liegt in der ersten Messkammer ein mittlerer Abstand von der Membran zu der ersten Elektrode zwischen 0,01 mm und 0,1 mm. Vorzugsweise liegt in der zweiten Messkammer und/oder der dritten Messkammer ein mittlerer Abstand von der Membran zu der zweiten Elektrode zwischen 0,01 mm und 0,1 mm

Vorzugsweise beträgt ein Volumen der ersten Messkammer zwischen 0,01 µL und 4 µL. Vorzugsweise beträgt ein Volumen der zweiten Messkammer und/oder der dritten Messkammer jeweils zwischen 0,01 µL und 4 µL. Es hat sich gezeigt, dass mit diesen Volumina besonders gute Messergebnisse bei unterschiedlichen Anströmgeschwindigkeiten erhalten werden können.

Eine bevorzugte Ausgestaltung sieht vor, dass die erste Messkammer, die zweite Messkammer und die dritte Messkammer durch Abschnitte der Verbindungsschicht voneinander getrennt sind. Auf diese Weise kann ein Vermischen der Flüssigkeiten in den Messkammern vermieden werden.

Vorzugsweise ist die Membran porös, insbesondere mikroporös. Damit ist sie in gewissem Umfang durchlässig für die Flüssigkeit und in der Flüssigkeit gelöste Stoffe. Auf diese Weise kann eine kleine Menge der zu messenden Flüssigkeit in die jeweilige Messkammer und damit in Kontakt mit den Elektroden gelangen. Verschmutzungen werden von der Membran von den Elektroden ferngehalten.

Erfindungsgemäß kann die Membran eine Folie aus Kunststoff umfassen.

Eine weitere Verbesserung sieht vor, dass die Membran hydrophil ist. Hierfür kann die Membran ein hydrophiles Material umfassen oder hydrophil ausgerüstet oder beschichtet sein.

In bevorzugter Weise kann die Membran als mikroporöse Filtermembran ausgebildet sein.

In bevorzugter Weise umfasst die Membran wenigstens einen Stoff ausgewählt aus der Gruppe, bestehend aus Polytetrafluorethylen (PTFE), Polycarbonat, Polyester, Polysulfon, Polyethersulfon, Polyvinylidenfluorid (PVDF), Polypropylen und Polyethylen.

Eine bevorzugte Ausgestaltung der Erfindung sieht vor, dass die Membran Poren mit einer Porengröße zwischen 0,1 µm und 10 µm (vorzugsweise 0,2 - 5 µm) umfasst. Damit können mit dem Sensor besonders gute Messergebnisse erreicht werden.

Erfindungsgemäß ist bevorzugt, dass die Membran eine Membrandicke zwischen 5 µm und 300 µm, vorzugsweise zwischen 6 µm und 50 µm, aufweist.

Eine bevorzugte Ausgestaltung der Erfindung sieht vor, dass die Verbindungsschicht die Membran an dem Träger fixiert.

Erfindungsgemäß kann vorgesehen sein, dass die Verbindungsschicht eine separate Schicht ist.

Vorzugsweise weist die Verbindungsschicht ein anderes Material als die Membran und als der Träger auf.

Erfindungsgemäß kann vorgesehen sein, dass die Verbindungsschicht flüssigkeitsdicht ist. Hierzu kann vorgesehen sein, dass die Verbindungsschicht keine Poren, insbesondere keine offenen Poren, aufweist.

Zudem kann vorgesehen sein, dass die Verbindungsschicht elektrisch nicht leitend ist.

Vorzugsweise bildet die Verbindungsschicht eine Isolierschicht.

Vorzugsweise enthält die Verbindungsschicht ein Polymer. Dabei ist bevorzugt, wenn es sich um ein vernetztes Polymer handelt.

Erfindungsgemäß kann vorgesehen sein, dass die Verbindungsschicht ein vernetztes Kunstharz aufweist. Vorzugsweise umfasst die Verbindungsschicht ein vernetztes Epoxid-Kunstharz.

Vorzugsweise bildet die Verbindungsschicht eine Klebschicht, die auf einer Seite klebend mit dem Träger und auf einer anderen Seite klebend mit der Membran verbunden ist. Das ermöglicht eine besonders gute Fixierung der Membran.

Eine vorteilhafte Ausführungsform sieht vor, dass die Verbindungsschicht eine Verbindungsschichtdicke aufweist, die der Dicke der ersten Elektrode entspricht. Bei dieser Ausführungsform kann die jeweilige Elektrode bereits ausreichend mit der Flüssigkeit benetzt werden, um zuverlässige Messungen durchzuführen.

Eine bevorzugte Ausführungsform sieht vor, dass die Verbindungsschicht eine Verbindungsschichtdicke aufweist, die größer ist als die Dicke der ersten Elektrode. Auf diese Weise schließt die jeweilige Messkammer einen definierten Raum über der jeweiligen Elektrode ein. Insbesondere kann die Verbindungsschichtdicke zwischen 0,01 mm und 0,1 mm größer sein als die Dicke der ersten Elektrode.

Eine bevorzugte Ausführungsform der Erfindung sieht vor, dass der Träger ein elektrisch nicht-leitendes Substrat umfasst. Vorzugsweise umfasst der Träger ein Keramikmaterial und/oder Kunststoffmaterial.

In vorteilhafter Weise kann vorgesehen sein, dass der Träger flach ist. Vorzugsweise ist der Träger plattenförmig ausgebildet.

Eine besonders bevorzugte Ausführungsform sieht vor, dass der Träger eine Rauheit Ra zwischen 0,1 µm und 2 µm, vorzugsweise zwischen 0,5 µm und 0,7µm, aufweist. Vorzugsweise weist der Träger die vorgenannte Rauheit in dem Flächenbereich auf, in dem die Elektroden angeordnet werden. Die Rauheit kann nach der Norm ISO 4287:2010 ermittelt werden. Mit diesen Rauheitswerten können die Elektroden besonders gut in einem Druckverfahren auf den Träger aufgebracht werden.

In bevorzugter Weise kann vorgesehen sein, dass der Träger in einem Sensorhalter aufgenommen ist.

Eine weitere Verbesserung wird dadurch erreicht, dass der Träger an dem Sensorhalter durch eine flüssigkeitsdichte Vergussmasse fixiert ist.

Die Vergussmasse kann ein vernetztes Kunstharz, insbesondere ein vernetztes Epoxidharz umfassen.

Vorzugsweise weist das Material der Vergussmasse vor dem Verfestigen eine Viskosität (bei 23°C und bei 50 rpm) zwischen 100 und 20.000 mPas (vorzugsweise zwischen 3.000 und 5.000 mPas) auf. Die niedrige Viskosität ist vorteilhaft für einen luftfreien Verguss.

Eine besonders bevorzugte Ausgestaltung sieht vor, dass der Sensorhalter einen Grundkörper aufweist, an dem wenigstens eine vorstehende Nase angeordnet ist. Vorzugsweise sind mehrere Nasen (besonders bevorzugt: zwei Nasen) vorgesehen, zwischen denen der Sensor angeordnet ist. Dabei kann vorgesehen sein, dass der Träger nicht gegenüber der Nase oder den Nasen vorsteht. Vorzugsweise bildet die Nase einen Aufsetzschutz, der den Sensor schützt. Zwischen den Nasen kann eine Durchflusszone für die Flüssigkeit ausgebildet sein.

Vorzugsweise ist eine erste elektrische Zuleitung für die erste Elektrode vorgesehen. Zudem können eine zweite elektrische Zuleitung für die zweite Elektrode und eine dritte elektrische Zuleitung für die dritte Elektrode vorgesehen sein.

Vorzugsweise ist auf dem Träger ein Kontakt zur Verbindung der ersten Elektrode mit der ersten Zuleitung angeordnet. Auf dem Träger können zudem Kontakte zur Verbindung der zweiten Elektrode mit der zweiten Zuleitung und der dritten Elektrode mit der dritten Zuleitung vorgesehen sein.

Vorzugsweise sind die erste, zweite und dritte elektrische Zuleitung mit einem lösbaren elektrischen Verbinder versehen. Das ermöglicht es, den Sensor auf einfache Weise auszutauschen. Der Verbinder kann insbesondere als Steckverbinder ausgebildet sein.

Die Erfindung bezieht sich auch auf die Verwendung des beschriebenen Sensors in einer Messeinrichtung zum Erfassen der Konzentration eines in einer Flüssigkeit enthaltenden Stoffes. Insbesondere bezieht sich die Erfindung auf die Verwendung des beschriebenen Sensors in einer Messeinrichtung zum Erfassen einer Konzentration eines chlorhaltigen Stoffes in einer wässrigen Flüssigkeit. Besonders bevorzugt ist die Verwendung des beschriebenen Sensors in einer Messeinrichtung zum Erfassen einer Konzentration wenigstens eines Stoffes ausgewählt aus der Liste, bestehend aus freiem Chlor, Chloraminen, Chlordioxid, Ozon, Wasserstoffperoxid, Brom und Chlorit in wässriger Flüssigkeit.

Die Erfindung bezieht sich auch auf eine Messeinrichtung zum Erfassen der Konzentration eines in einer Flüssigkeit enthaltenen Stoffes, umfassend einen Sensor der beschriebenen Art.

Die Messeinrichtung kann insbesondere eine amperometrische Messeinrichtung sein.

Vorzugsweise ist der Sensor in einer Durchflusszone der Messeinrichtung angeordnet.

In bevorzugter Weise ist die Messeinrichtung eine Einrichtung zum Messen einer Konzentration eines chlorhaltigen Stoffes in einer wässrigen Flüssigkeit. Besonders bevorzugt ist die Messeinrichtung eine Einrichtung zum Messen einer Konzentration wenigstens eines Stoffes ausgewählt aus der Liste, bestehend aus freiem Chlor, Chloraminen, Chlordioxid, Ozon, Wasserstoffperoxid, Brom und Chlorit in wässriger Flüssigkeit.

Erfindungsgemäß kann vorgesehen sein, dass die Messeinrichtung eine Steuer- und Auswerteeinheit, die zumindest mit der ersten und der zweiten Elektrode verbunden ist, umfasst. Vorzugsweise umfasst die Steuer- und Auswerteeinheit einen Potentiostaten. Ein Potentiostat kann als elektronischer Regelverstärker ausgeführt sein, mit dem das Potential, d. h. die Spannung der ersten Elektrode, auf einen gewünschten Wert geregelt wird.

Eine bevorzugte Ausführung sieht vor, dass die Steuer- und Auswerteeinheit ein Amperometer umfasst. Mit dem Amperometer kann der die erste Elektrode durchfließende Strom gemessen werden.

Vorzugsweise weist die Messeinrichtung ein Gehäuse auf, an dem der Träger befestigt ist, wobei ein Abschnitt des Trägers mit zumindest einem Teil der ersten Elektrode aus dem Gehäuse herausragt.

Das Gehäuse bildet eine Messzone, die von wenigstens einem Gehäuseabschnitt begrenzt wird, wobei der Träger in der Messzone angeordnet ist.

Die Erfindung stellt sich auch die Aufgabe, ein Verfahren zur einfachen und sicheren Herstellung eines Sensors anzugeben.

Erfindungsgemäß kann das Verfahren zur Herstellung des Sensors folgende Schritte aufweisen:
- Bereitstellen eines Trägers;
- Aufbringen der ersten Elektrode auf den Träger;
- Aufbringen der Verbindungsschicht, wobei die Verbindungsschicht in dem Bereich der ersten Elektrode eine erste Aussparung aufweist, um eine erste Messkammer zu bilden;
- Aufbringen der Membran auf die Verbindungsschicht.

Auf diese Weise kann der vorliegend beschriebene Sensor mit hoher Prozesssicherheit und kostengünstig hergestellt werden.

Vorzugsweise wird das Material der ersten Elektrode als eine Polymermasse, die ein Metall enthält, auf den Träger aufgebracht und anschließend verfestigt. Bei Verwendung einer thermoplastischen Polymermasse kann diese erwärmt und durch Abkühlen verfestigt werden. Bevorzugt ist eine vernetzbare Polymermasse, die nach dem Aufbringen auf den Träger durch Vernetzung verfestigt wird.

Weiterhin kann vorgesehen sein, dass das Material der zweiten Elektrode als eine verfestigbare Polymermasse, die ein Metall enthält, auf den Träger aufgebracht und anschließend verfestigt wird. Wenn der Sensor eine dritte Elektrode umfasst, kann das Material der dritten Elektrode als eine verfestigbare Polymermasse, die ein Metall enthält, auf den Träger aufgebracht und anschließend verfestigt sein. Auch für die zweite und dritte Elektrode ist bevorzugt, dass die verfestigbare Polymermasse eine vernetzbare Polymermasse ist, die nach dem Aufbringen auf den Träger durch Vernetzung verfestigt wird.

Eine weitere Verbesserung wird dann erreicht, wenn das Material der Verbindungsschicht als eine verfestigbare Polymermasse auf den Träger aufgebracht wird. Das Material der Verbindungsschicht kann mit der ersten Aussparung, der zweiten Aussparung und der dritten Aussparung aufgebracht werden. Bevorzugt ist für die Verbindungsschicht eine vernetzbare Polymermasse, die nach dem Aufbringen auf den Träger durch Vernetzung verfestigt wird.

Vorzugsweise erfolgt das Aufbringen des Materials der Verbindungsschicht, nachdem das Material der ersten Elektrode (und ggf. der zweiten Elektrode und ggf. der dritten Elektrode) ausgehärtet wurde.

Eine besonders bevorzugte Ausgestaltung sieht vor, dass die Membran auf die Polymermasse der Verbindungsschicht aufgebracht wird, bevor die Polymermasse der Verbindungsschicht vernetzt wird.

Erfindungsgemäß ist bevorzugt, dass das Material der ersten Elektrode und/oder das Material der zweiten Elektrode und/oder das Material der dritten Elektrode und/oder das Material der Verbindungsschicht durch Drucken aufgebracht werden. Drucken mit Siebdruck ist dabei besonders bevorzugt. Auf diese Weise können die jeweiligen Elektroden in der gewünschten Form und Größe hergestellt werden. Bei der Verbindungsschicht ermöglicht die Herstellung durch Drucken, auf einfache Weise die Aussparungen für die Messkammern herzustellen.

Vorzugsweise weist die Polymermasse der Verbindungsschicht beim Aufbringen auf den Träger eine Viskosität (bei 23°C und 2,5 rpm) zwischen 30.000 und 200.000 mPas (vorzugsweise zwischen 80.000 und 120.000 mPas) auf. Die hohe Viskosität ist vorteilhaft, damit die die Messkammer bildenden Aussparungen erhalten bleiben, wenn die Membran aufgebracht wird.

Weitere Ziele, Merkmale, Vorteile und Anwendungsmöglichkeiten der vorliegenden Erfindung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger sinnvoller Kombination den Gegenstand der Erfindung, auch unabhängig von der Zusammenfassung in einzelnen Ansprüchen oder deren Rückbeziehungen.

Es zeigen:
- Fig. 1:: eine schematische Darstellung einer Messeinrichtung mit einem elektrochemischen Sensor;
- Fig. 2:: eine Seitenansicht des elektrochemischen Sensors aus Fig. 1;
- Fig. 3:: eine Messeinrichtung mit einem Sensorhalter, der einen Sensor aufweist, in perspektivischer Darstellung;
- Fig. 4a:: den Sensorhalter für die Messeinrichtung in Fig. 3 ohne Sensor;
- Fig. 4b:: den Sensorhalter aus Fig. 4a mit Sensor.

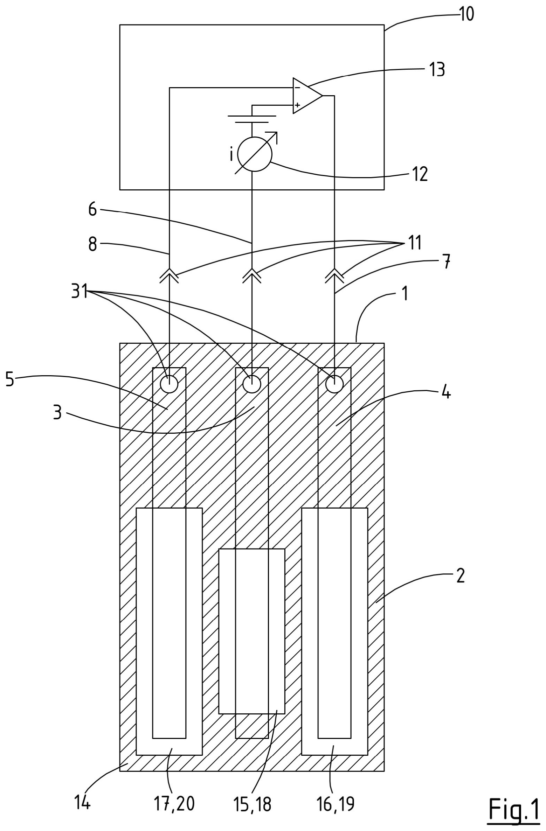

**Fig. 1** zeigt einen elektrochemischen Sensor 1 mit einem Träger 2, auf dem eine erste Elektrode 3, eine zweite Elektrode 4 und eine dritte Elektrode 5 angeordnet sind. Der Sensor 1 ist Teil einer schematisch dargestellten Messeinrichtung.

Im dargestellten Ausführungsbeispiel sind die erste, zweite und dritte Elektrode 3, 4, 5 länglich und streifenförmig ausgebildet.

Die erste Elektrode 3 bildet eine Arbeitselektrode für die Messeinrichtung und enthält ein Metall, wie insbesondere Gold, Platin oder Palladium.

Die zweite Elektrode 4 bildet eine Gegenelektrode für die Messeinrichtung. Diese enthält insbesondere ein Metall, wie Gold, Platin oder Palladium.

Die dritte Elektrode 5 bildet eine Bezugselektrode für die Messeinrichtung. Diese enthält ein anderes Metall als die Arbeitselektrode, wie z. B. Silber und/oder ein Silberhalogenid.

Der Träger 2 umfasst ein elektrisch nicht-leitendes Substrat. Er kann insbesondere aus einem Keramikmaterial und/oder einem Kunststoffmaterial gebildet sein. Der Träger 2 ist plattenförmig ausgebildet und weist im dargestellten Ausführungsbeispiel eine rechteckige Grundform auf.

**Fig. 1** zeigt neben dem Sensor 1 weitere Elemente einer Messeinrichtung zum Erfassen der Konzentration eines in einer Flüssigkeit enthaltenen Stoffes. Schematisch dargestellt ist dabei eine Steuer- und Auswerteinheit 10, die über elektrische Zuleitungen 6, 7 und 8 mit der ersten Elektrode 3, der zweiten Elektrode 4 und der dritten Elektrode 5 verbunden ist. Die Verbindung erfolgt dabei über lösbare elektrische Verbinder 11, die als Steckverbinder ausgebildet sein können. Auf diese Weise kann der Sensor 1 auf einfache Weise ausgetauscht werden.

Auf dem Träger 2 sind für jede Elektrode 3, 4, 5 ein Kontakt 31 zur Verbindung der jeweiligen Elektrode 3, 4, 5 mit ihrer Zuleitung 6, 7, 8 vorgesehen.

Mit der Steuer- und Auswerteeinheit 10 kann an der ersten Elektrode 3 ein Arbeitspotential erzeugt und eingestellt werden, sodass in einer Flüssigkeit enthaltende Chlormoleküle an der Elektrodenoberfläche Elektronen aufnehmen und zu zwei Chloridionen reduziert werden. Dem liegt folgende vereinfachte Reaktionsgleichung zugrunde:

Cl₂ + 2e → 2 Cl⁻

Der dabei entstehende Strom kann mit einem Amperometer 12 der Steuer- und Auswerteeinheit 10 gemessen werden.

Die Steuer- und Auswerteeinheit 10 weist einen Potentiostaten 13 auf, der den elektrochemischen Prozess steuert. Das Amperometer 12 ermittelt den dabei entstehenden Strom. Das Stromsignal kann analog temperaturkorrigiert und als analoges elektrisches Signal an Mess- und Regelgeräte ausgegeben werden. Eine andere Möglichkeit ist, dass das Stromsignal digitalisiert wird, um digital temperaturkorrigiert und an digitale Mess- und Regelgeräte ausgegeben zu werden. Das Arbeitspotential des Sensors 1 kann geändert werden, um eine Anpassung an die Chloridionenkonzentration in der Flüssigkeit vorzunehmen.

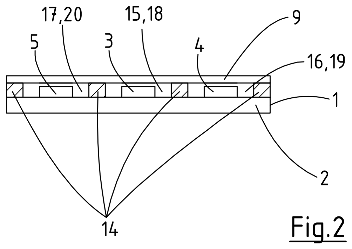

**Fig. 2** zeigt genauer den Aufbau des Sensors 1. Hier sind in Seitenansicht die erste, zweite und dritte Elektrode 3, 4, 5 zu erkennen, die auf dem Träger 2 angeordnet sind. Zudem zeigt **Fig. 2****,** dass der Sensor eine Membran 9 aufweist. Diese überdeckt die erste Elektrode 3, die zweite Elektrode 4 und die dritte Elektrode 5. Die Membran 9 ist über eine Verbindungsschicht 14 mit dem Träger 2 verbunden. Die Verbindungsschicht 14 weist in dem Bereich der ersten Elektrode 3 eine erste Aussparung 15 auf. Im Bereich der zweiten Elektrode 4 ist eine zweite Aussparung 16 und im Bereich der dritten Elektrode 5 eine dritte Aussparung 17 der Verbindungsschicht 14 vorgesehen. Auf diese Weise wird über der ersten Elektrode 3 eine erste Messkammer 18 gebildet. Über der zweiten Elektrode 4 wird eine zweite Messkammer 19 und über der dritten Elektrode 5 eine dritte Messkammer 20 gebildet. Die erste, die zweite und die dritte Messkammer 18, 19, 20 sind gegeneinander durch die Verbindungsschicht 14 abgedichtet, sodass kein direkter Austausch von Flüssigkeit zwischen den Messkammern erfolgt.

Die Ausgestaltung der Verbindungsschicht 14 ist in **Fig. 1** genauer dargestellt. Die Figur zeigt in Schraffur die Anordnung der Verbindungsschicht 14. Ohne Schraffur sind die in der Verbindungsschicht 14 ausgebildeten ersten, zweiten und dritten Aussparungen 15,16 und 17 dargestellt.

Die erste Aussparung 15 lässt nur einen Teil der ersten Elektrode 3 frei, sodass dieser in Kontakt mit der zu messenden Flüssigkeit gelangen kann. Das wird dadurch erreicht, dass ein Teil der ersten Elektrode 3 durch die Verbindungsschicht 14 abgedeckt ist. Auch die zweite Aussparung 16 lässt nur einen Teil der zweiten Elektrode 4 frei. Entsprechendes gilt auch für die dritte Aussparung 17, die nur ein Teil der dritten Elektrode 5 frei lässt.

Auf diese Weise sind eine erste, zweite und dritte Messkammer 18, 19, 20 gebildet, die insbesondere eine Fläche zwischen 1 mm² und 40 mm² aufweisen können. Auf diese Weise kann die aktive Fläche der ersten, zweiten und dritten Elektrode 3, 4, 5 definiert werden.

Im dargestellten Ausführungsbeispiel ist die erste Aussparung 15 kleiner als die zweite und die dritte Aussparung 16, 17. Auf diese Weise kann die aktive Fläche der ersten Elektrode 3 kleiner definiert werden als die aktiven Flächen der zweiten und der dritten Elektroden 4, 5.

**Fig. 2** zeigt, dass die Verbindungsschicht 14 eine Verbindungsschichtdicke aufweist, die größer ist als die Dicke der ersten, zweiten und dritten Elektrode 3, 4, 5. Dadurch ergibt sich ein Abstand zwischen der Membran 9 und der ersten, zweiten und dritten Elektrode 3, 4, 5. Auf diese Weise ergeben sich erste, zweite und dritte Messkammern 18, 19, 20, die jeweils ein definiertes Messvolumen aufweisen. Erfindungsgemäß hat sich bewährt, wenn ein Volumen der ersten, zweiten und dritten Messkammer 18, 19, 20 jeweils zwischen 0,01 µl und 4 µl beträgt.

Die Membran 9 ist mikroporös. Damit ist sie für die Flüssigkeit, insbesondere Wasser, in dem die Messung erfolgen soll, durchlässig. Gleichzeitig hält die mikroporöse Struktur der Membran 9 Verschmutzungen von der ersten, zweiten und dritten Elektrode 3, 4, 5 fern. Eine für den Sensor 1 geeignete mikroporöse Membran ist beispielsweise von der Firma Merck unter dem Produktnamen Millipore Express PLUS Membran Typ GPWP04700 erhältlich.

Die Membran 9 kann durch eine Folie aus Kunststoff gebildet sein, die Poren aufweist. Erfindungsgemäß hat sich bewährt, wenn die Poren eine Porengröße zwischen 0,1 µm und 10 µm, bevorzugt 0,2 µm bis 5 µm, aufweisen.

Die in **Fig. 2** nicht maßstabsgerecht dargestellte Membran 9 weist eine Membrandicke zwischen 5 µm und 300 µm, vorzugsweise zwischen 6 µm und 50 µm, auf.

Um den dosierten Durchtritt einer wässrigen Flüssigkeit durch die Membran zu erleichtern, kann die Membran hydrophil sein. Hierzu kann das Material der Membran 9 selbst hydrophil sein. Ein Beispiel für ein hydrophiles Material ist Polyethersulfon. Die Membran 9 kann aber auch andere Kunststoffe enthalten. Sofern deren Material an sich nicht hydrophil ist, kann dieses hydrophil ausgerüstet oder beschichtet sein.

Die Verbindungsschicht 14 fixiert die Membran 9 an dem Träger 2. Die Verbindungsschicht 14 ist als eine separate Schicht ausgebildet. Die Verbindungsschicht weist keine offenen Poren auf. Die Verbindungsschicht 14 verhindert, dass Flüssigkeit zwischen der ersten, zweiten und dritten Messkammer 18, 19, 20 ausgetauscht wird.

Die Verbindungsschicht 14 kann insbesondere eine vernetzte Polymermasse enthalten. Die Verbindungsschicht 14 bildet eine Klebschicht, die auf einer Seite klebend mit dem Träger 2 und auf der gegenüberliegenden Seite klebend mit der Membran 9 verbunden ist.

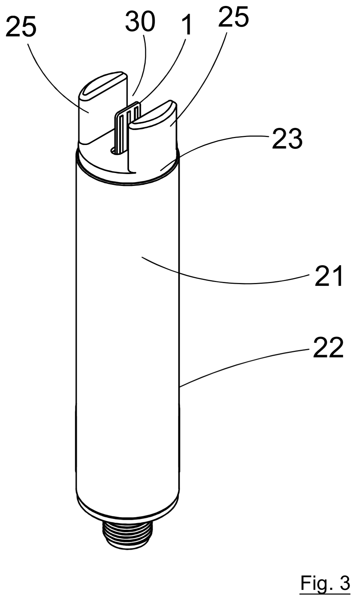

**Fig. 3** zeigt eine perspektivische Darstellung einer Messeinrichtung 21 zum Erfassen der Konzentration eines in einer Flüssigkeit enthaltenen Stoffes. Die Messeinrichtung 21 weist ein Gehäuse 22 auf, in dem die in **Fig. 1** dargestellte Steuer- und Auswerteeinheit 10 angeordnet ist. Die Messeinrichtung 21 umfasst darüber hinaus einen Halter 23, an dem der Sensor 1 befestigt ist. Der Sensor 1 ragt dabei aus dem Halter 23 heraus, sodass der Sensor 1 direkt in der zu messenden Flüssigkeit angeordnet werden kann. Dabei ist der Sensor 1 in einer Durchflusszone 30 der Messeinrichtung angeordnet.

Der Halter 23 weist einen Grundkörper 24 auf, an dem zwei vorstehende Nasen 25 angeordnet sind. Der Träger 2 des Sensors 1 ist dabei zwischen den zwei Nasen 25 angeordnet. Der Sensor 1 steht dabei nicht gegenüber den Nasen 25 vor. Auf diese Weise bilden die Nasen 25 einen Anstoßschutz, die den Sensor 1 vor mechanischer Beschädigung schützen. Gleichzeitig wird eine ungestörte Anströmung des Sensors ermöglicht.

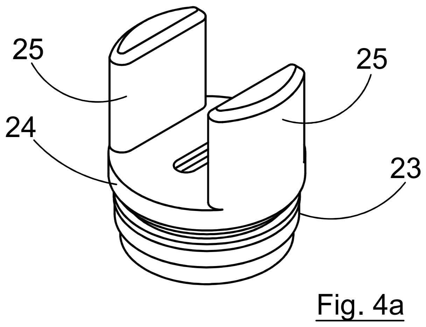

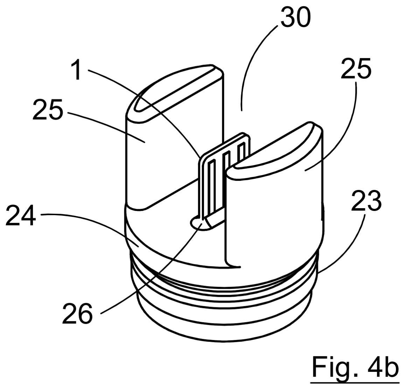

**Fig. 4a** zeigt schematisch einen Halter 23 für die Messeinrichtung 21. Der Halter 23 kann abnehmbar an dem Gehäuse 22 aus **Fig. 3** befestigt werden. Auf diese Weise kann der Sensor 1 mit dem Halter 23 leicht ausgewechselt werden. **Fig. 4a** zeigt den Halter 23 ohne den Sensor 1. In **Fig. 4b** ist der Halter 23 mit dem Sensor 1 dargestellt. Der Sensor 1 kann in dem Halter durch eine Vergussmasse 26 fixiert werden. Insbesondere kann die Vergussmasse 26 ein vernetztes Kunstharz, wie insbesondere ein vernetztes Epoxidharz, sein.

Die Vergussmasse weist vor dem Vernetzen vorzugsweise eine Viskosität (bei 23 °C und bei 50 rpm) auf, die zwischen 100 und 20.000 mPas liegt.

Die Messeinrichtung 21 kann in eine nicht dargestellte Durchflussarmatur eingesetzt werden.

Wenn der Sensor 1 in Kontakt mit der zu messenden Flüssigkeit gebracht wird, dann füllen sich die anfangs leeren ersten, zweiten und dritten Messkammern 18, 19, 20 mit der Flüssigkeit. Die Flüssigkeit fungiert als Elektrolyt für die Messung.

Nachfolgend wird ein Verfahren zur Herstellung des in den **Fig. 1** und **2** dargestellten Sensors beschrieben.

Zunächst wird ein Träger 2 bereitgestellt. Dieser kann insbesondere aus Kunststoff oder Keramik sein. Der Träger 2 kann in dem Bereich, in dem die Elektroden und die Verbindungsschicht 14 aufgebracht werden, eine Rauheit Ra zwischen 0,1 µm und 2 µm, vorzugsweise zwischen 0,5 µm und 0,7µm, aufweisen.

Anschließend werden die erste, zweite und dritte Elektrode 3, 4, 5 aufgebracht. Für das Aufbringen kommen unterschiedliche Beschichtungsverfahren in Betracht. Beispielsweise kann zur Bildung der Elektroden ein Metall durch Kathodenzerstäubung (sogenanntes Sputtern) auf den Träger aufgebracht werden. In Betracht kommt darüber hinaus die physikalische Gasphasenabscheidung, auch PVD-Verfahren genannt. Zudem kann ein metallisches Material im Ionenstrahlverfahren aufgebracht werden.

Erfindungsgemäß besonders bevorzugt ist das Aufbringen des Elektrodenmaterials als eine Paste oder Tinte aus polymerem Material. Die hierbei verwendeten Pasten oder Tinten können das für die jeweilige Elektrode gewünschte Metall, wie beispielsweise Gold, Silber, Platin, Palladium, Ruthenium, Tantal oder deren Oxide und/oder andere geeignete Metalle, Metalloxide oder Mischungen von diesen, aufweisen. Darüber hinaus können die Pasten/Tinten auch weitere Beimischungen von funktionswichtigen Substanzen, wie z. B. Silberhalogeniden, wie Silberchlorid, Silberjodid und/oder Silberbromid, enthalten. Die Pasten können insbesondere durch Siebdruck, Rolle-zu-Rolle-Verfahren, Jetting oder Tampondruck aufgebracht werden. Mit diesen Verfahren können die erste, zweite und dritte Elektrode 3, 4, 5 in der gewünschten Form und Größe auf dem Träger 2 angeordnet werden. Bei der Herstellung im Siebdruck können Siebe verwendet werden, die Drucköffnungen entsprechend den gewünschten Elektroden aufweisen.

Die Paste oder Tinte kann insbesondere ein Polymer aufweisen, dass nach dem Aufbringen verfestigt wird. Insbesondere geeignet sind vernetzbare Polymere, die nach dem Aufbringen auf den Träger 2 vernetzt werden.

Für die Herstellung der Elektroden geeignete verfestigbare Polymermassen sind von dem Hersteller Nanochemazone^{™} unter den Produktbezeichnungen Platinum Paste und Silver/Silver Chloride Paste erhältlich.

Auch die Verbindungsschicht 14 kann mit einem Druckverfahren gezielt in der gewünschten Form und Größe und insbesondere mit den beschriebenen ersten, zweiten und dritten Aussparungen 15, 16, 17 hergestellt werden. Bei der Herstellung im Siebdruck kann ein Sieb verwendet werden, das eine Drucköffnung entsprechend der gewünschten Form der Verbindungsschicht 14 aufweist. Auch für die Verbindungsschicht 14 kann eine Polymermasse verwendet werden, die vernetzt werden kann. Insbesondere kommt für die Verbindungsschicht 14 eine vernetzbare Polymermasse, wie ein Epoxidharz, in Betracht. Dieses kann mit den oben beschriebenen Techniken auf den Träger 2 aufgebracht werden. Wie in **Fig. 1** dargestellt, werden dabei von der Verbindungsschicht 14 Abschnitte der ersten, zweiten und dritten Elektrode 3, 4, 5 überdeckt. Andere Bereiche der ersten, zweiten und dritten Elektroden 3, 4 und 5 bleiben aufgrund der Aussparungen 15, 16 und 17 in der Verbindungsschicht 14 frei.

Nach dem Aufbringen des Materials der Verbindungsschicht 14 wird die Membran 9 aufgebracht. Die Membran 9, die insbesondere eine Folie sein kann, wird dabei auf das Material der Verbindungsschicht gelegt und ggf. leicht angedrückt. Erst nach dem Aufbringen der Membran 9 wird das Material der Verbindungsschicht 14 verfestigt und insbesondere vernetzt. Hierdurch wird die Membran 9 sicher an dem Träger 2 klebend befestigt.

Hierbei hat sich bewährt, wenn das Material der Verbindungsschicht 14 vor dem Verfestigen eine Viskosität (bei 23 °C und 2,5 rpm) zwischen 30.000 und 200.000 mPas aufweist. Mit dem genannten Viskositätsbereich wird erreicht, dass das Material der Verbindungsschicht vor und beim Aufbringen der Membran 9 so stabil ist, dass die angestrebte Form der Verbindungsschicht 14 mit der ersten, zweiten und dritten Aussparungen 15, 16, 17 in klar abgegrenzter Form erhalten werden kann.

Nach dem Aufbringen der Membran 9 und dem Verfestigen der Verbindungsschicht 14 kann der fertige Sensor 1 in den Halter 23 eingesetzt werden und mit einer Vergussmasse 26 an diesem befestigt werden. Die wasserdichte Vergussmasse 26 verhindert ein Eintreten der Flüssigkeit in den Halter 23 und damit in das Gehäuse 22.

Die beschriebene Messeinrichtung 21 ist besonders geeignet für die amperometrische Messung einer Konzentration eines chlorhaltigen Stoffes in einer wässrigen Flüssigkeit. Insbesondere ist die Messeinrichtung geeignet für die amperometrische Messung der Konzentration wenigstens eines Stoffes ausgewählt aus der Liste, bestehend aus freiem Chlor, Chloraminen, Chlordioxid, Ozon, Wasserstoffperoxid, Brom und Chlorit.

## Patentansprüche

1. Elektrochemischer Sensor mit einer ersten Elektrode (3) und einer zweiten Elektrode (4), mit einem Träger (2), auf dem zumindest die erste Elektrode (3) angeordnet ist, und mit einer Poren aufweisenden Membran (9), **dadurch gekennzeichnet, dass** die Membran (9) an dem Träger (2) angeordnet ist und die erste Elektrode (3) überdecket, wobei die Membran (9) über eine Verbindungsschicht (14) mit dem Träger (2) verbunden ist, wobei die Verbindungsschicht (14) in dem Bereich der ersten Elektrode (3) eine erste Aussparung (15) aufweist, um über der ersten Elektrode (3) eine erste Messkammer (18) zu bilden.

2. Elektrochemischer Sensor nach Anspruch 1, **dadurch gekennzeichnet, dass** auf dem Träger (2) die zweite Elektrode (4) angeordnet ist, wobei die Membran (9) die zweite Elektrode (4) überdeckt, wobei die Verbindungsschicht (14) in dem Bereich der zweiten Elektrode (4) eine zweite Aussparung (16) aufweist, um über der zweiten Elektrode (4) eine zweite Messkammer (19) zu bilden

3. Elektrochemischer Sensor nach Anspruch 2, **dadurch gekennzeichnet, dass** die erste und die zweite Messkammer (18, 19) gegeneinander abgedichtet sind.

4. Elektrochemischer Sensor nach Anspruch 2 oder 3, **dadurch gekennzeichnet, dass** auf dem Träger (2) eine dritte Elektrode (5) angeordnet ist, wobei die Membran (9) die dritte Elektrode (5) überdeckt, wobei die Verbindungsschicht (14) in dem Bereich der dritten Elektrode (5) eine dritte Aussparung (17) aufweist, um über der dritten Elektrode (5) eine dritte Messkammer (20) zu bilden.

5. Elektrochemischer Sensor nach einem der Ansprüche 1 bis 4, **dadurch gekennzeichnet, dass** die erste Aussparung (15) nur einen Teil der ersten Elektrode (3) frei lässt.

6. Elektrochemischer Sensor nach einem der Ansprüche 1 bis 5, **dadurch gekennzeichnet, dass** ein Volumen der ersten Messkammer (18) zwischen 0,01 und 4 µL beträgt.

7. Elektrochemischer Sensor nach einem der Ansprüche 1 bis 6, **dadurch gekennzeichnet, dass** die Membran Poren mit einer Porengröße zwischen 0,1 und 10 µm umfasst.

8. Elektrochemischer Sensor nach einem der Ansprüche 1 bis 7, **dadurch gekennzeichnet, dass** die Verbindungsschicht (14) eine Klebschicht bildet, die auf einer Seite klebend mit dem Träger (2) und auf einer anderen Seite klebend mit der Membran (9) verbunden ist.

9. Elektrochemischer Sensor nach einem der Ansprüche 1 bis 8, **dadurch gekennzeichnet, dass** der Träger eine Rauheit Ra zwischen 0,1 und 2 µm aufweist.

10. Elektrochemischer Sensor nach einem der Ansprüche 1 bis 9, **dadurch gekennzeichnet, dass** der Träger (2) in einem Sensorhalter (23) aufgenommen ist.

11. Amperometrische Messeinrichtung zum Erfassen der Konzentration eines in einer Flüssigkeit enthaltenen Stoffes, umfassend einen elektrochemischen Sensor (1) nach einem der Ansprüche 1 bis 10.

12. Verfahren zur Herstellung eines elektrochemischen Sensors nach einem der Ansprüche 1 bis 10, mit folgenden Schritten:
- Bereitstellen des Trägers (2);
- Aufbringen der ersten Elektrode (3) auf den Träger (2);
- Aufbringen der Verbindungsschicht (14) auf den Träger, wobei die Verbindungsschicht (14) in dem Bereich der ersten Elektrode (3) eine erste Aussparung (15) aufweist, um eine erste Messkammer (18) zu bilden.
- Aufbringen der Membran (9) auf die Verbindungsschicht (14).

13. Verfahren nach Anspruch 12, **dadurch gekennzeichnet, dass** das Material der ersten Elektrode (3) als eine verfestigbare Polymermasse, die ein Metall enthält, auf den Träger (2) aufgebracht und verfestigt wird.

14. Verfahren nach Anspruch 12 oder 13, **dadurch gekennzeichnet, dass** das Material der Verbindungsschicht (14) als eine verfestigbare Polymermasse auf den Träger (2) aufgebracht wird, wobei im Bereich der ersten Messkammer (18) kein Material der Verbindungsschicht aufgebracht wird.

15. Verfahren nach Anspruch 14, **dadurch gekennzeichnet, dass** die Membran (9) auf das Material der Verbindungsschicht (14) aufgebracht wird, bevor die Polymermasse der Verbindungsschicht (14) verfestigt wird.
